# EUROPEAN PATENT SPECIFICATION

(11) **EP 2 010 471 B1**
(45) Date of publication and mention of the grant of the patent: **12.10.2011**
(21) Application number: 07723911.9
(22) Date of filing: 03.04.2007
(51) Int. Cl.: C07C 37/08, C07C 37/72, C07C 37/82, C07C 37/68, C07C 39/04

(54) **PROCESS FOR REMOVAL OF HYDROXYACETONE FROM PHENOL**
VERFAHREN ZUM ENTFERNEN VON HYDROXYACETON AUS PHENOL
PROCEDE D'ELIMINATION D'HYDROXYACETONE DU PHENOL

(30) Priority: 18.04.2006 EP 06007957
(43) Date of publication of application: 07.01.2009
(73) Proprietor: INEOS Phenol GmbH & Co. KG, 45966 Gladbeck (DE)
(72) Inventor: WEBER, Manfred, 45721 Haltern (DE); WEBER, Markus, 45721 Haltern (DE); SCHNURR, Otto, B-2950 Putte-Kapellen (BE)
(74) Representative: Polypatent
(86) International application number: PCT/EP2007/002970
(87) International publication number: WO 2007/118601

(56) References cited:
- DD-A- 73 326
- US-A- 3 029 294
- US-A- 4 283 568
- US-A- 5 414 154
- US-B2- 6 875 898
- VASILEVA I I ET AL: "NEFTECHIMIJA, PASSAGE" NEFTEPERERABATYVAUSAA I NEFTEHIMICESKAA PROMYSENNOST SERIA, CNIITENEFTEHIM, MOSCOW, RU, no. 12, 2000, pages 34-38, XP001098357 ISSN: 0236-1418 cited in the application

## Description

The present invention relates to a method for producing phenol, particularly to a method wherein the product obtained from the acid catalyzed cleavage of cumene hydroperoxide is separated by distillation into at least a phenol-containing fraction and an aqueous fraction comprising hydroxyacetone whereby said aqueous fraction is treated with an oxidizing agent in presence of a base to obtain a basic aqueous medium reduced in hydroxyacetone.

### Background of the invention

The process for preparing phenol from cumene is well known. In this process cumene is at first oxidized by air oxygen to cumene hydroperoxide. This process step is typically called oxidation. In the second reaction step, the so-called cleavage, the cumene hydroperoxide is cleaved to phenol and acetone using a strong mineral acid as catalyst, for example sulfuric acid. The product from this second reaction step, the so-called cleavage product, is then fractionated by distillation.

The purity requirements for phenol to be marketed are becoming more and more stringent. Consequently, in order to operate a phenol production plant economically, overall yield and selectivity to the desired end product has to be improved and impurities formed during any of the above-described reaction steps have to be removed as quantitatively as possible with the lowest possible loss of the desired end product, especially phenol and acetone, at low investment and variable costs, especially energy costs. The predominant by-products formed in the oxidation steps are dimethylbenzyl alcohol and acetophenone. Acetophenone leaves the process with the high-boilers from the distillation. Dimethylbenzyl alcohol is dehydrated in the cleavage step to alpha-methylstyrene which partially forms high-boiling dimers and cumylphenols in the acid catalyst cleavage step. The high-boilers are separated from phenol in the distillation step. The unreacted alpha-methylstyrene is separated and hydrogenated in order to form cumene that is recycled into the process. Depending on the market demand, alpha-methylstyrene can also be further purified and sold as value product. Thus, one focus in the prior art is how to operate the oxidation step as well as the cleavage step in order to reduce the formation of these high-boilers which can be considered as direct cumene losses. For example for the cleavage these methods are described in US-A-4,358,618, US-A-5,254,751, WO98/27039 and US-6,555,719.

But besides these high-boilers other components are formed in the cleavage, as for example hydroxyacetone, 2-methylbenzofuran and mesityloxide. These so-called micro impurities are not easy to separate from phenol in the distillation. Hydroxyacetone is the most critical component as it is nearly impossible to separate it from phenol by simple distillation. Hydroxyacetone is typically also the micro-impurity with the highest concentration in the product obtained from the cleavage step. The concentration of hydroxyacetone in the cleavage product may vary between 200 and 3.000 wppm (weight parts per million).

Thus, there are great efforts in the prior art to remove and separate hydroxyacetone from the product obtained from the cleavage step (see for example US 6,066,767, US 6,630,608, US 6,576,798 and US 6,875,898). The disadvantage of all these methods is that high volume flows of cleavage product must be processed. In addition, in US 6,875,898, the high volume flow of cleavage product must be treated with an oxidizing agent that may cause enormous efforts to operate the process safely.

Prior to distillation, the cleavage product is neutralized with a basic aqueous solution such as sodium phenate or caustic soda. The cleavage product which is saturated with water is then worked up by distillation. A well known method is to separate most of the hydroxyacetone with an aqueous phase which is separated in the first distillation column while a crude phenol together with the high-boilers is taken as the bottom product, as described in US 3,405,038 or in US 6,657,087.

According to the teaching of US 6,657,087, a portion of the aqueous phase obtained after phase separation of the side take-off product of the first distillation column is discarded whereas the remainder is returned to the first distillation column. Consequently, discarded portion of the aqueous phase has to be subjected to waste water treatment according to safety and environmental legislation. This considerably increases the costs of running the process. Furthermore, the portion of water discarded from the system has to be reintroduced as fresh water which is additionally a waste of resources. Thus, it is the object of the present invention to provide a method for producing phenol that avoids the disadvantages of the prior art discussed above and allows for an effective reduction of hydroxyacetone from the crude phenol stream at low investment and variable costs.

US patent 4,283,568 discloses a process for the recovery of phenol from a reaction mixture resulting from the acid cleavage of cumene hydroperoxide. Neutralization of the acidic reaction mixture is effected with sodium phenate - a product derived from the subsequent recovery of phenol and recycled to the neutralization process.

### Summary of the invention

This object has been attained by a method for producing phenol comprising:
a) oxidizing cumene to form an oxidation product containing cumene hydroperoxide;
b) cleaving said oxidation product using an acidic catalyst to form a cleavage product containing phenol, acetone and impurities;
c) neutralizing and washing said cleavage product with a basic aqueous medium to obtain a neutralized cleavage product;
d) separating said neutralized cleavage product by at least one distillation step into at least a phenol containing fraction and an aqueous fraction comprising hydroxyacetone;
e) treating said aqueous fraction with an oxidizing agent in presence of a base to obtain a basic aqueous medium reduced in hydroxyacetone;
f) recycling at least a portion of said basic aqueous medium to the neutralizing and washing step c); and
g) recovering phenol from said phenol containing fraction obtained in step d).

Compared to the disclosure of US 6,576,798, only a low volume aqueous stream, comprising hydroxyacetone obtained from the distillative separation of the cleavage product, has to be treated with an oxidizing agent. Furthermore, compared to the experimental data presented in US 6,576,798, the residual amount of hydroxyacetone in the crude phenol stream is considerably reduced when using the process of the present invention. Furthermore, compared to the teaching of US 6,657,087, no hydroxyacetone containing aqueous stream obtained from the distillation of the cleavage products that has to be subjected to waste water treatment is discarded without compromising the quality of the crude phenol in terms of residual hydroxyacetone.

### Detailed description of the present invention

According to the present invention, most of the hydroxyacetone present in the cleavage product obtained from the acid catalyzed cleavage of cumene hydroperoxide, preferably more than 90 percent, is removed with an aqueous fraction obtained by separating the neutralized cleavage product by at least one distillation step. The aqueous phase comprising the hydroxyacetone removed from the cleavage product is treated with an oxidizing agent in presence of a base. Thereby, hydroxyacetone is converted into neutralized oxidation products, for example salts of the corresponding carboxyl-functional material, resulting in a basic aqueous medium having a reduced hydroxyacetone content. At least a portion of said basic aqueous medium is used for neutralizing the cleavage product.

According to a preferred embodiment, the aqueous fraction treated with an oxidizing agent in presence of a base is completely recycled to the step of neutralizing and washing the cleavage product. Thereby any additional waste water stream obtained when separating the neutralized cleavage product by distillation is avoided.

According to a preferred embodiment, in the neutralization and washing step c) the mixture of cleavage product and aqueous medium is heterogeneous and after the neutralizing washing step c) and prior to the separation step d) the heterogeneous mixture is phase separated into an aqueous phase containing at least a part of the neutralized oxidation products of hydroxyacetone and any additional salts from the neutralization of the cleavage product and a water-saturated organic phase that is fed to the separation step d).

Consequently, any salt material including the neutralized oxidation products from hydroxyacetone is discharged from the system in a single waste water stream after neutralization of the cleavage product.

According to a preferred embodiment, the base is added to the aqueous fraction obtained from the work-up of the neutralized cleavage product prior to the oxidizing treatment. Preferably, the base is added in an amount to adjust the pH to be greater than 8, preferably to be between 10 and 12. Any water-soluble base can be used according to the present invention, but is preferred if the base is selected from aqueous NaOH and aqueous phenoxide solutions. It is particularly preferred to use an aqueous sodium phenate solution whereby the concentration of sodium phenate of the sodium phenate solution is preferably 5 to 50, more preferred 30 to 45 and most preferred 40 to 45 percent by weight. Such sodium phenate solutions are generally obtained as process stream in a standard phenol plant as result of one or a plurality of subsequent work-up steps. The use of a process stream anyway obtained in a phenol plant has the advantage that neither fresh water nor fresh caustic has to be introduced into the process step of the present invention. Furthermore, phenol lost as sodium phenate in the work-up steps of a phenol plant is thereby recycled into the process so that loss of valuable material is minimized.

The oxidizing agent to be used according to the process of the present invention can be any suitable oxidizing agent that is capable of converting hydroperoxide into oxidation products. Preferably, the oxidizing agent is selected from hydrogen peroxide, oxygen, air or any mixture of oxygen and nitrogen, whereby air is most preferred. The oxygen-containing gas, for example air, may be either dissolved in the aqueous fraction obtained from the separating step d), as explained above, by keeping the system under a sufficiently high pressure, or the oxygen-containing gas is dispersed in the liquid in a gas liquid reactor. Both alternatives of reacting an aqueous phase with a gaseous oxidizing agent are well-known in the prior art.

The temperature for treating said aqueous fraction with an oxidizing agent may vary between 20 and 150°C, preferably between 80 and 120°C, and more preferred between 90 and 110°C. Preferably, the temperature and residence time in the treating step is adjusted in order to convert at least 90 percent of the hydroxyacetone present in the aqueous phase obtained from the separation step into neutralized oxidation products of hydroxyacetone.

The aqueous phase obtained from the separation step d) of the present invention may contain residual amounts of acetone and phenol, but it was surprisingly found that none of these components react during the oxidation of hydroxyacetone resulting in undesired side products. Due to the recycle of the aqueous phase in to the phenol process, any unwanted loss of valuable products, like acetone and phenol, is avoided despite the oxidation step. According to a preferred embodiment of the present invention, the acetone content is less than 0.1 wt.-% , preferably in the wppm range. This can be achieved by sufficiently high separation efficiency in the separating step d) or by treating the aqueous phase prior to oxidation in an acetone stripper. With such a low acetone content no problems arise when contacting the aqueous phase with any kind of oxidizing agent because any gaseous phase will be non-explosive. This is one of the most important advantages compared to the methods described in US 6,576,798 and in Vasileva, I.I et al. 2000, Neftepereab. Neftekhim. Moscow, Russ. Fed., 12:34-38.

Another important advantage of the present invention is that compared to the methods described in US 6,066,767, US 6,630,608, US 6,576,798 and US 6875,898 only a relatively small volume flow of an aqueous phase must be treated, thus keeping the reactor volume necessary for the treating step e) small resulting in low investment costs.

Typically, depending on the hydroxyacetone content in the cleavage product, the crude phenol obtained from the separation step d) of the present invention contains between 50 and 400 wppm hydroxyacetone. It is preferred to further treat the crude phenol stream obtained from the separation step d) to further reduce the content of hydroxyacetone and other impurities.

Thus according to a preferred embodiment of the present invention a crude phenol stream comprising methylbenzofuran and hydroxyacetone is treated in a continuous method by passing the crude phenol stream through at least two reactors connected in series the reactors containing an acidic ion exchange resin, whereby the temperature in successive reactors decreases in flow direction of the phenol stream so that the temperature in the first reactor in flow direction of the phenol stream is between 100°C and 200°C and the temperature in the last reactor in flow direction of the phenol stream is between 50°C and 90°C without a thermal separation step between any of two successive reactors.

The present inventors have realized that by using a plurality of reactors containing the acidic ion exchange resin in series and importantly adjusting a temperature profile throughout the series of reactors as defined above a crude phenol stream can be purified to a low content of hydroxyacetone as well as methylbenzofuran without initially removing hydroxyacetone prior to contact with the acidic ion exchange resin and without an energy-consuming distillation step between two reactors comprising the acidic ion exchange resin. Furthermore surprisingly, although at least two reactors have to be used, the overall weight hourly space velocity of the process according to the this preferred embodiment is considerably higher than for the one-step process described in US 2005/0137429 with the effect that the total reactor volume required according to the present invention is even lower than for the one-step process, as disclosed in US 2005/0137429.

The process for treating a crude phenol stream can be easily integrated into the process of the resent invention.

The crude phenol that can be effectively purified by the treating step of the preferred embodiment of the present invention contains as impurities predominantly hydroxyacetone as well as methylbenzofuran. The concentration of hydroxyacetone can be up to 1,000 wppm and the concentration of methylbenzofuran can be up to 200 ppm. One advantage of the present invention is that hydroxyacetone as well as methylbenzofuran can be effectively removed even if the hydroxyacetone concentration is more than 260 wppm. Thus, a crude phenol stream comprising up to 1,000 wppm, preferably more than 260 wppm to 1,000 wppm hydroxyacetone and up to 200 wppm, preferably 50 to 200 wppm methylbenzofuran can be successfully purified.

In addition to hydroxyacetone and methylbenzofuran further impurities may be present:
Mesityloxide up to 1,000 wppm,
2-phenylpropionaldehyde up to 500 wppm,
methylisobutylketone up to 500 wppm,
acetophenone up to 500 wppm,
3-methylcyclohexanone up to 500 wppm,
alpha-methylstyrene up to 2,000 wppm,
phenylbutenes up to 1,000 wppm.

These concentration ranges cover the relevant concentrations of these components in crude phenol which is separated from acetone, cumene and alpha-methylstyrene, water and high-boilers by distillation prior to the purification on an ion exchange resin.

When contacting the crude phenol stream with the acidic ion exchange resin hydroxyacetone and methylbenzofuran react to high-boilers. Mesityloxide reacts with phenol to high-boilers and water. In the presence of water, which is also formed by the reaction between hydroxyacetone and phenol, parts of the mesityloxide may decompose to acetone on the acidic ion exchange resin. Acetone may further react with phenol to Bisphenol A. Besides hydroxyacetone and mesityloxide there are other carbonylic components which may still be present in the phenol in small amounts, like phenylpropionaldehyde, methylisobutylketone, acetophenone and 3-methylcyclohexanone. In addition, the phenol may have final traces of unsaturated hydrocarbons, like alpha-methylstyrene and phenolbutenes which are undesirable components in purified phenol. Like the carbonyl-containing components, the unsaturated hydrocarbons form high-boilers with phenol when in contact with acidic ion exchange resins. It was found that, even if these other impurities are present in impure phenol, the conversion of hydroxyacetone and methylbenzofuran is not adversely effected. Furthermore, the conversion of these additional impurity components to high-boilers is always completed when the conversion of hydroxyacetone and methylbenzofuran is completed. Consequently, the process of the present invention allows for the conversion of all the undesired impurities in crude phenol to high-boilers that can be easily removed from the purified phenol in a final distillation step after the crude phenol has been contacted with the acidic ion exchange resin according to the process according to the present invention.

After contact of the crude phenol with the acidic ion exchange resin, final concentration of hydroxyacetone of less than 1 wppm and concentrations of methylbenzofuran of less than 20 wppm, preferably less than 10 wppm, can be obtained. As mentioned above, all other impurities are quantitatively converted to high-boilers. Therefore, the process according to the present invention is well suited to prepare high purity phenol. The number of reactors containing the acidic ion exchange resin connected in series and, thus, the number of different temperature levels according to the present invention is not particularly restricted, but taking into account economic considerations in terms of investment costs and variable costs, a number of two to four reactors connected in series is preferred whereby two reactors connected in series are most preferred. Thus, according to this most preferred embodiment, the process is conducted at two distinguished temperature levels.

Furthermore, the present inventors have found that the deactivation of commercial ion exchange resin correlates very well with the degree of utilization. The degree of utilization is defined as the total amount of treated phenol which was contacted with the ion exchange resin during a certain period of time. For a continuous plug flow reactor this is the total amount of treated phenol per cross-sectional area of the reactor.

After a high degree of utilization the activity of the catalyst is only some percent of that of the fresh catalyst. Surprisingly the temperature, that is necessary to compensate the deactivation at a constant weight hourly space velocity (WHSV), increases proportional to the degree of utilization. From practical considerations the maximal temperature is 200°C in order to avoid any thermal degradation of commercial ion exchange resins.

On the other hand it was found that for a phenol stream comprising methylbenzofuran as well as considerably amounts of hydroxyacetone e.g. up to 200 wppm methylbenzofuran and up to 1000 wppm hydroxyacetone a temperature in the last reactor below 90°C is necessary to obtain a residual amount of methylbenzofuran below 20 wppm, preferably below 70°C to obtain a residual amount of methylbenzofuran below 10 wppm. From practical considerations the temperature should not be below 50°C in order to avoid a too high reactor volume even with fresh catalyst.

One advantage of having a plurality of distinct temperature levels for the contact of crude phenol with the acidic ion exchange resin is that used or partly used acidic ion exchange resin can be contacted at relatively high temperatures that for example favor the conversion of hydroxyacetone, but not the conversion of methylbenzofuran , with the result that even with a used or partly used catalyst due to the high temperatures a high activity of the already spent catalyst can be maintained. On the other hand, at the low temperature level fresh or only partly used catalyst can be employed at low temperatures favoring the conversion of methylbenzofuran and since the catalyst is still relatively fresh, high catalyst activity can be obtained even at low temperatures. Consequently, an optimum balance of selectivity of the contact with the acidic ion exchange resin can be obtained while at the same time assuring optimum activity of the catalyst resulting in comparatively high weight hourly space velocity thereby reducing the necessary catalyst volume for treatment of a specific phenol stream.

This synergistic effect of optimization of catalyst selectivity with respect to hydroxyacetone and methylbenzofuran and catalyst activity depending on the grade of deactivation of the catalyst by using the claimed temperature profile was neither known nor derivable from the prior art.

A further advantage of the preferred embodiment of the present invention is that if several reactors are connected in series, including at least one spare reactor, in a continuous process completely spent catalyst can be easily removed from the process line. The reactor with the most spent catalyst which is at the highest temperature level and, thus, at the upstream end can be disconnected from the line, and the reactor with fresh catalyst will enter the line at the lowest temperature level, thus at the downstream end of the line. In the reactor that is disconnected from the line, the spent catalyst will be either substituted by fresh catalyst or regenerated in a separate process step in order to retain the initial activity of the fresh catalyst. This reactivated reactor can then enter the line at the lowest temperature level as soon as the reactor at the highest temperature level, wherein the catalyst has been deactivated to an undesirable level, is removed from the line. This allows for a continuous process wherein the efficiency of the purification is approximately constant over the time resulting in a product of almost constant specification which is extremely important for a high volume product as phenol.

It is preferred to use reactors of the same size. Thus, at each position in the line, the WHSV for a certain phenol stream is the same and does not change while changing the positions of the reactors in the line. The necessary temperatures in the reactors with ion exchange resins of different activities can easily be determined.

Furthermore, a plurality of reactors connected in parallel can be used for every temperature level. Thus, it is very easy to adapt the treating process to a changing throughput. Again it is preferred to use reactors of the same size and the same number of reactors at each temperature level.

Additionally, it is possible to use a heat integration of the phenol stream going through the reactors in order to minimize energy consumption. For example, the phenol stream can be passed through a heat exchanger between a first reactor and a successive second reactor using a colder phenol effluent from a reactor located downstream from the first reactor as coolant in the heat exchanger. This embodiment allows to cooling down the phenol stream between two successive reactors whereas at the same time the phenol stream leaving the last reactor at the lowest temperature level, when used as a coolant in the heat exchanger, is heated up so that the energy consumption in the subsequent distillation step to remove the high-boilers is reduced.

Furthermore, additional heat exchangers can be used between two successive reactors employing conventional coolants like cooling water to adjust the temperature of the phenol stream to the desired level.

According to one embodiment of the present invention, elongated vessels are used as reactors whereby the vessels are preferably arranged in a vertical orientation whereby the phenol flows from the top to the bottom of the reactor. But it is also possible to use an upstream flow in vertical vessels or to use horizontal vessels.

According to a preferred embodiment of the present invention, the reactors contain the acidic ion exchange resin in a fixed bed. Preferably, the superficial liquid velocity in the fixed bed of the ion exchange resin is 0.5 to 5 mm/sec, preferable 1.0 to 3.0 mm/sec and more preferred 1.5 to 2 mm/sec.

Any acidic ion exchange resin can be used as the catalyst according to the present invention. As used herein, the term "acidic ion exchange resin" refers to a cation exchange resin in the hydrogen form wherein the hydrogen ions are bound to the active sides which can be removed either by dissociation in solution or by replacement with other positive ions. The active sides of the resins have different attractive strengths for different ions and this selective attraction serves as means for ion exchange. Non-limiting examples of suitable acidic ion exchange resins include the series of sulfonated divinylbenzene crosslinked styrene copolymers, such as for example Amberlyst 16, commercially available from Rohm & Haas, K2431, commercially available from Lanxess, CT-151, commercially available from Purolite.

Other suitable resins can be commercially obtained from producers such as Lanxess, Rohm and Haas Chemical Company and Dow Chemical Company.

According to the preferred embodiment of the present invention, the temperature in the first reactor in flow direction of the phenol stream is at least 100°C and temperature of the last reactor in flow direction of the phenol stream is less than 90 °C, preferably less than 70°C.

The temperature in the first reactor in flow direction of the phenol stream is 200°C at most, preferably 150°C at most, and most preferred 120°C at most. The temperature in the last reactor in flow direction of the phenol stream is at least 50°C.

The present invention will now be further illustrated with reference to a specific embodiment and examples.

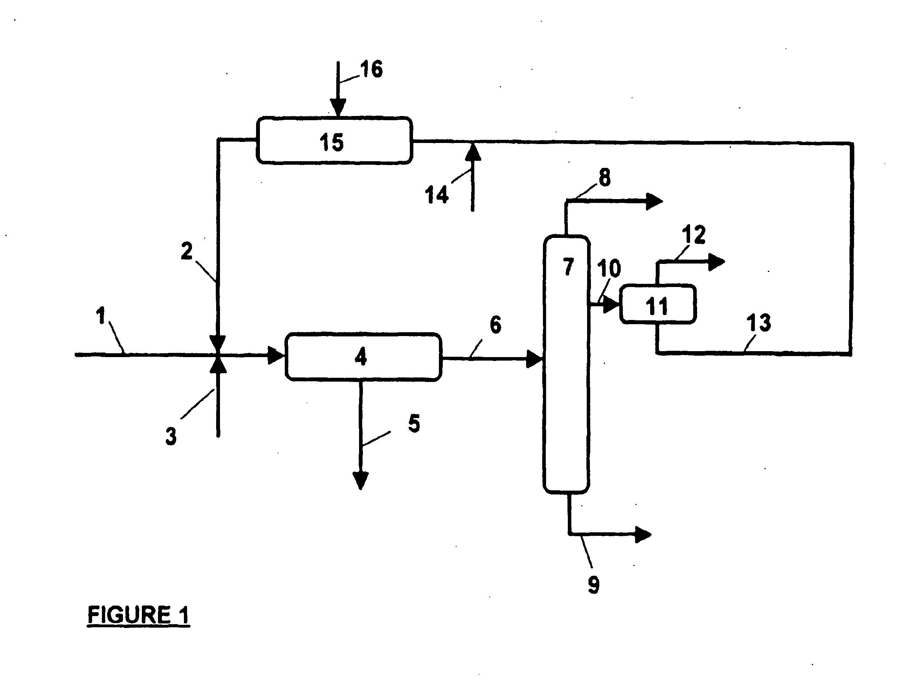

According to the embodiment shown in Fig. 1, the cleavage product obtained from the acid catalyzed cleavage of cumene hydroperoxide is fed via line 1 to a settler drum 4. Prior to entry of the cleavage product into the settler drum the cleavage product is mixed with an aqueous phase containing the salts from the oxidation products from hydroxyacetone and excess base, preferably sodium phenate, which is fed to the system via line 2. If necessary, sulfuric acid may be added via line 3 in order to adjust the pH typically to a range between 4 to 8. The resulting mixture is heterogeneous and is separated in the settler drum 4 into an aqueous phase 5 containing salts from the oxidation products of hydroxyacetone as well as sodium sulfate, sodium formiate and salts from other organic acids, and into an organic phase saturated with water. The aqueous phase, comprising in addition to the above-mentioned salts small amounts of hydroxyacetone, is withdrawn for further treatment via line 5. The water-saturated organic phase is fed to distillation column 7 via line 6. In distillation column 7 the organic phase is separated into a crude phenol fraction removed from the bottom of the distillation column 7 via line 9, a crude acetone fraction removed from the head of the column 7 via line 8, and a fraction comprising water, cumene, alpha-methylstyrene and most of the hydroxyacetone removed from the distillation column at a side take-off. Said aqueous phase is fed via line 10 to a settler drum 11 whereby the fraction is separated into an aqueous phase comprising hydroxyacetone and an organic phase comprising cumene and alpha-methylstyrene. The organic phase is fed via line 12 to subsequent work-up steps. The aqueous phase comprises, besides hydroxyacetone, small amounts of acetone, preferably less than 0.1 wt.-%, some phenol as well as some organic acids like formic acid or acetic acid and is mixed with a base introduced via line 14 in order to increase the pH to be above 8, preferably between 10 and 12. According to a preferred embodiment, an aqueous sodium phenate solution within the concentration ranges discussed above is used. Preferably, the mixing ratio of aqueous phase obtained from the distillation column 7 and the aqueous sodium phenate solution is in the range of 1:0.05 to 1:1, preferably between 1:0.1 to 1:0.3. Thereby a homogeneous mixture of aqueous phase and sodium phenate solution is obtained that is fed to the reactor 15. An oxidizing agent, preferably air, is introduced into the reactor via line 16 and temperature and residence time in the reactor are adjusted in order to convert at least 90 percent of the hydroxyacetone into the corresponding neutralized oxidation products. The hydroxyacetone content in the aqueous phase obtained from the distillation step is typically between 0.5 and 2 wt.-% . The effluent from the reactor 15 containing the salts from the oxidation products from hydroxyacetone, excess sodium phenate and residual amounts of hydroxyacetone is then, as discussed above, used to neutralize the cleavage product.

Alternatively to the process described with reference to Fig. 1, it is also possible to operate the first column without any side take-off to get an overhead product comprising acetone, cumene, alpha-methylstyrene, water and most of the hydroxyacetone. This overhead product can then be separated in a subsequent pure acetone column, as shown in US 5,510,543, to obtain a bottom product comprising cumene, water and hydroxyacetone. This bottom product can then be separated in a settler drum and treated, as discussed above with reference to Fig. 1.

### Examples

### Comparative example:

A cleavage product contains 42 wt.-% phenol, 26 wt.-% acetone, 25 wt.-% cumene, 3.1 wt.-% alpha-methylstyrene, 200 wppm dissolved sulfuric acid and 1500 wppm hydroxyacetone besides other organic components. All concentrations are related to the total amount of organic components (water-free).
In addition, 1 wt.-% dissolved water is present. 10 wt.-% additional fresh water, related to the total amount of cleavage product, must be added to the cleavage product to saturate the cleavage product with water and to form an aqueous phase containing salts. The salts are coming from neutralization by adding a sufficient amount of sodium sulphate to adjust the pH in the aqueous phase to around 6. In the first distillation column water is taken as a side draw containing 90 % of the hydroxyacetone resulting in a concentration of about 1.23 wt.-% of hydroxyacetone in the water. The crude phenol as the bottom product contains 340 wppm of hydroxyacetone. The water is withdrawn from the process and sent to further treatment, amongst others towards biological treatment.

### Example:

The cleavage product of the comparative example is mixed with water from the oxidation reactor of hydroxyacetone. The water contains 0.1 wt.-% of residual hydroxyacetone and sodium phenate. The resulting concentration of hydroxyacetone in the cleavage product is around 1590 wppm. Some sulfuric acid is added to adjust the pH to around 6. In the first distillation column water is taken as a side draw containing 90 % of the hydroxyacetone resulting in a concentration of about 1.30 wt.-% of hydroxyacetone in the water. The crude phenol as the bottom product contains 360 wppm of hydroxyacetone. The water is mixed with a 40 wt.-% aqueous sodium phenate solution in a weight ratio of 1:0.1 and contacted with pure oxygen at 95°C. The conversion rate of hydroxyacetone is 92 % thus resulting in a residual concentration of hydroxyacetone of 0.1 wt.-%. The water is completely recycled to the neutralization of the cleavage product.

From the comparison of the comparative example and the example of the present invention it is evident that an additional waste water stream is avoided without compromising the quality of the crude phenol stream in terms of hydroxyacetone concentration.

## Claims

1. A method for producing phenol comprising:
a) oxidizing cumene to form an oxidation product containing cumene hydroperoxide;
b) cleaving said oxidation product using an acidic catalyst to form a cleavage product containing phenol, acetone and impurities;
c) neutralizing and washing said cleavage product with a basic aqueous medium to obtain a neutralized cleavage product;
d) separating said neutralized cleavage product by at least one distillation step into at least a phenol containing fraction and an aqueous fraction comprising hydroxyacetone;
e) treating said aqueous fraction with an oxidizing agent in presence of a base to obtain a basic aqueous medium reduced in hydroxyacetone;
f) recycling at least a portion of said basic aqueous medium to the neutralizing and washing step c); and
g) recovering phenol from said phenol containing fraction obtained in step d).

2. The method of claim 1, wherein in step e) said base is added to the aqueous fraction prior to the oxidizing treatment.

3. The method of claim 2, wherein the base is added in an amount to adjust the pH to be greater than 8, preferably to be between 10 and 12.

4. The method of any of the preceding claims, wherein in step e) said base is an aqueous sodium phenate solution.

5. The method of claim 4, wherein the concentration of sodium phenate in the sodium phenate solution is 5 to 50, preferably 30 to 45, more preferred 40 to 45 percent by weight.

6. The method of claim 1, wherein in the treating step e) hydroxyacetone is converted into neutralized oxidation products
preferably at least 90% of the hydroxyacetone is converted into neutralized oxidation products, by adjusting temperature and residence time,

7. The method of any of the preceding claims, wherein the acetone content of said aqueous fraction is less than 0.1 weight percent.

8. The method of any of the preceding claims, wherein the temperature in the treating step e) is 20 - 150°C, preferably 80 to 120°C, more preferred 90 to 110°C.

9. The method of any of the preceding claims, wherein in the neutralization and washing step c) the mixture of cleavage product and aqueous medium is heterogeneous and after the neutralization and washing step c) and prior to the separation step d) the heterogeneous mixture is phase-separated into an aqueous phase containing at least a part of the neutralized oxidation products of hydroxyacetone and a water saturated organic phase that is fed to the separation step d).

10. The method of any of the preceding claims, wherein said aqueous fraction obtained in the separation step d) comprises more than 90% of the hydroxyacetone present in the neutralized cleavage product fed to the separation step d).

11. The method of any of the preceding claims, wherein the crude phenol obtained from separation step d) comprises methylbenzofuran and hydroxyacetone and is treated by passing the crude phenol stream through at least two reactors connected in series the reactors containing an acidic ion exchange resin, whereby the temperature in successive reactors decreases in flow direction of the phenol stream so that the temperature in the first reactor in flow direction of the phenol stream is between 100°C and 200°C and the temperature in the last reactor in flow direction of the phenol stream is between 50°C and 90°C without a thermal separation step between any of two successive reactors.

12. The method of claim 11, wherein 2 to 4 reactors connected in series are employed.

13. The method of any of claims 11 or 12, wherein the temperature in the first reactor in flow direction of the phenol stream is between 100°C and 150°C. preferably between 100°C and 120°C and the temperature in the last reactor in flow direction of the phenol stream is between 50°C and 70°C.

14. The method of any of claims 11-13, wherein the reactors contain the acid ion exchange resin in fixed bed arrangement.

15. The method of any of claims 11-14, wherein the phenol stream is passed through an heat exchanger between a first reactor and a successive second reactor using a colder phenol effluent from a reactor located downstream from the first reactor as coolant in the heat exchanger.

## Patentansprüche

1. Verfahren zur Herstellung von Phenol, umfassend:
a) Oxidieren von Cumol, um ein Oxidationsprodukt zu bilden, das Cumolhydroperoxid enthält,
b) Spalten dieses Oxidationsprodukts unter Verwendung eines sauren Katalysators, um ein Spaltprodukt zu bilden, das Phenol, Aceton und Verunreinigungen enthält,
c) Neutralisieren und Waschen dieses Spaltprodukts mit einem basischen wässrigen Medium, um ein neutralisiertes Spaltprodukt zu erhalten,
d) Auftrennen dieses neutralisierten Spaltprodukts mit wenigstens einem Destillationsschritt in wenigstens eine phenolhaltige Fraktion und eine wässrige Fraktion, die Hydroxyaceton enthält,
e) Behandeln dieser wässrigen Fraktion mit einem Oxidationsmittel in Gegenwart einer Base, um ein basisches wässriges Medium mit reduziertem Hydroxyacetongehalt zu erhalten,
f) Zurückführen wenigstens eines Teils dieses basischen wässrigen Mediums zu dem Neutralisations- und Waschschritt c) und
g) Gewinnen von Phenol aus dieser phenolhaltigen Fraktion, die in Schritt d) erhalten wird.

2. Verfahren nach Anspruch 1, wobei in Schritt e) diese Base zu der wässrigen Fraktion vor der oxidativen Behandlung zugegeben wird.

3. Verfahren nach Anspruch 2, wobei die Base in einer Menge zugegeben wird, um den pH auf größer als 8, vorzugsweise zwischen 10 und 12 einzustellen.

4. Verfahren nach einem der vorstehenden Ansprüche, wobei in Schritt e) diese Base eine wässrige Natriumphenatlösung ist.

5. Verfahren nach Anspruch 4, wobei die Konzentration an Natriumphenat in der Natriumphenatlösung 5 bis 50, vorzugsweise 30 bis 45, besonders bevorzugt 40 bis 45 Gew.-% beträgt.

6. Verfahren nach Anspruch 1, wobei in dem Behandlungsschritt e) Hydroxyaceton in neutralisierte Oxidationsprodukte, vorzugsweise wenigstens 90% des Hydroxyacetons in neutralisierte Oxidationsprodukte durch Einstellung der Temperatur und der Verweilzeit umgewandelt werden.

7. Verfahren nach einem der vorstehenden Ansprüche, wobei der Acetongehalt dieser wässrigen Fraktion weniger als 0,1 Gew.-% beträgt.

8. Verfahren nach einem der vorstehenden Ansprüche, wobei die Temperatur im Behandlungsschritt e) 20 bis 150°C, vorzugsweise 80 bis 120°C, besonders bevorzugt 90 bis 110°C beträgt.

9. Verfahren nach einem der vorstehenden Ansprüche, wobei in dem Neutralisations- und Waschschritt c) die Mischung des Spaltprodukts und des wässrigen Mediums heterogen ist und nach dem Neutralisations- und Waschschritt c) und vor dem Trennschritt d) die heterogene Mischung in eine wässrige Phase, die wenigstens einen Teil des neutralisierten Oxidationsprodukts von Hydroxyaceton enthält, und eine mit Wasser gesättigte organische Phase, die in den Trennschritt d) geführt wird, phasengetrennt wird.

10. Verfahren nach einem der vorstehenden Ansprüche, wobei diese wässrige Fraktion, die in Trennschritt d) erhalten wird, mehr als 90 % des Hydroxyacetons enthält, das in dem neutralisierten Spaltprodukt vorhanden ist, das zum Trennschritt d) geführt wird.

11. Verfahren nach einem der vorstehenden Ansprüche, wobei das Rohphenol, das aus dem Trennschritt d) erhalten wird, Methylbenzofuran und Hydroxyaceton enthält und durch Durchführen des Rohphenolstroms durch wenigstens zwei in Serie geschaltete Reaktoren behandelt wird, wobei die Reaktoren ein saures Ionenaustauscherharz enthalten und die Temperatur in aufeinanderfolgenden Reaktoren in Strömungsrichtung des Phenolstroms abnimmt, so dass die Temperatur in dem ersten Reaktor in Strömungsrichtung des Phenolstroms zwischen 100°C und 200°C liegt und die Temperatur in dem letzten Reaktor in Strömungsrichtung des Phenolstroms zwischen 50°C und 90°C liegt, ohne einen thermischen Trennschritt zwischen beliebigen zwei aufeinanderfolgenden Reaktoren.

12. Verfahren nach Anspruch 11, wobei 2 bis 4 in Serie geschaltete Reaktoren verwendet werden.

13. Verfahren nach einem der Ansprüche 11 oder 12, wobei die Temperatur in dem ersten Reaktor in Strömungsrichtung des Phenolstroms zwischen 100°C und 150°C liegt, vorzugsweise zwischen 100°C und 120°C, und die Temperatur in dem letzten Reaktor in Strömungsrichtung des Phenolstroms zwischen 50°C und 70°C liegt.

14. Verfahren nach einem der Ansprüche 11-13, wobei die Reaktoren das saure Ionenaustauscherharz in einer Festbettanordnung enthalten.

15. Verfahren nach einem der Ansprüche 11-14, wobei der Phenolstrom durch einen Wärmeaustauscher zwischen einem ersten Reaktor und einem darauffolgenden zweiten Reaktor unter Verwendung eines kälteren Phenolaustrittstroms aus einem Reaktor, der in Strömungsrichtung nach dem ersten Reaktor angeordnet ist, als Kühlmittel in den Wärmeaustauscher geleitet wird.

## Revendications

1. Procédé de production de phénol comprenant les étapes suivantes :
a) l'oxydation de cumène pour former un produit d'oxydation contenant de l'hydroperoxyde de cumène ;
b) le clivage dudit produit d'oxydation en utilisant un catalyseur acide de manière à former un produit de clivage contenant du phénol, de l'acétone et des impuretés ;
c) la neutralisation et le lavage dudit produit de clivage avec un milieu basique aqueux pour obtenir un produit de clivage neutralisé ;
d) la séparation dudit produit de clivage neutralisé via au moins une étape de distillation pour obtenir au moins une fraction contenant du phénol et une fraction aqueuse comprenant de l'hydroxyacétone ;
e) le traitement de ladite fraction aqueuse avec un agent oxydant en présence d'une base de manière à obtenir un milieu basique aqueux à moindre teneur en hydroxyacétone ;
f) le recyclage d'au moins une partie dudit milieu basique aqueux à l'étape de neutralisation et de lavage c) ; et
g) la récupération de phénol de ladite fraction contenant du phénol obtenue à l'étape d).

2. Procédé selon la revendication 1, dans lequel, à l'étape e), ladite base est ajoutée à la fraction aqueuse avant le traitement oxydant.

3. Procédé selon la revendication 2, dans lequel la base est ajoutée en quantité permettant d'ajuster le pH à une valeur supérieure à 8, de préférence, à une valeur comprise entre 10 et 12.

4. Procédé selon l'une quelconque des revendications précédentes, dans lequel, à l'étape e), ladite base est une solution aqueuse de phénate de sodium.

5. Procédé selon la revendication 4, dans lequel la concentration du phénate de sodium dans la solution de phénate de sodium est de 5 à 50, de préférence de 30 à 45, mieux encore, de 40 à 45 pour cent en poids.

6. Procédé selon la revendication 1, dans lequel, à l'étape de traitement e), l'hydroxyacétone est convertie en produits d'oxydation neutralisés, de préférence au moins 90 % de l'hydroxyacétone sont convertis en produits d'oxydation neutralisés, en ajustant la température et le temps de séjour.

7. Procédé selon l'une quelconque des revendications précédentes, dans lequel la teneur en acétone de ladite fraction aqueuse est inférieure à 0,1 pour cent en poids.

8. Procédé selon l'une quelconque des revendications précédentes, dans lequel la température à l'étape de traitement e) se situe dans la plage de 20 à 150 °C, de préférence de 80 à 120 °C, mieux encore de 90 à 110 °C.

9. Procédé selon l'une quelconque des revendications précédentes, dans lequel, à l'étape de neutralisation et de lavage c), le mélange du produit de clivage et du milieu aqueux est hétérogène et, après l'étape de neutralisation et de lavage c) et avant l'étape de séparation d), le mélange hétérogène est soumis à une partition de phases pour donner une phase aqueuse contenant au moins une partie des produits d'oxydation neutralisés de l'hydroxyacétone et une phase organique saturée en eau qui est délivrée à l'étape de séparation d).

10. Procédé selon l'une quelconque des revendications précédentes, dans lequel ladite fraction aqueuse obtenue à l'étape de séparation d) comprend plus que 90 % de l'hydroxyacétone présente dans le produit de clivage neutralisé délivré à l'étape de séparation d).

11. Procédé selon l'une quelconque des revendications précédentes, dans lequel le phénol brut obtenu à l'étape de séparation d) comprend du méthylbenzofuranne et de l'hydroxyacétone et est traité en faisant passer le courant de phénol brut à travers au moins deux réacteurs raccordés en série, les réacteurs contenant une résine d'échange ionique acide, moyennant quoi la température dans les réacteurs successifs diminue dans le sens de l'écoulement du courant de phénol de sorte que la température dans le premier réacteur dans le sens d'écoulement du courant de phénol se situe entre 100 °C et 200 °C et que la température dans le dernier réacteur dans le sens de l'écoulement du courant de phénol se situe entre 50 °C et 90 °C en l'absence d'une étape de séparation thermique entre deux réacteurs successifs quelconques.

12. Procédé selon la revendication 11, dans lequel on utilise 2 à 4 réacteurs raccordés en série.

13. Procédé selon l'une quelconque des revendications 11 ou 12, dans lequel la température dans le premier réacteur dans le sens d'écoulement du courant de phénol se situe entre 100 °C et 150 °C, de préférence entre 100 °C et 120 °C, et la température dans le dernier réacteur dans le sens d'écoulement du courant de phénol se situe entre 50 °C et 70 °C.

14. Procédé selon l'une quelconque des revendications 11 à 13, dans lequel les réacteurs contiennent la résine d'échange ionique acide selon un aménagement en lit fixe.

15. Procédé selon l'une quelconque des revendications 11 à 14, dans lequel on fait passer le courant de phénol à travers un échangeur de chaleur entre un premier réacteur et un second réacteur successif en utilisant un effluent de phénol plus froid provenant d'un réacteur situé en aval du premier réacteur comme réfrigérant dans l'échangeur de chaleur.
